(19) **Europäisches Patentamt / European Patent Office / Office européen des brevets**

(11) **EP 1 937 212 B1**

(12) **EUROPEAN PATENT SPECIFICATION**

(45) Date of publication and mention
of the grant of the patent:
**01.09.2010  Bulletin 2010/35**

(51) Int Cl.:
**A61K 9/107** *(2006.01)*    **A61K 31/5575** *(2006.01)*

(21) Application number: **06806156.3**

(86) International application number:
**PCT/EP2006/009783**

(22) Date of filing: **10.10.2006**

(87) International publication number:
**WO 2007/042262 (19.04.2007 Gazette 2007/16)**

(54) **OPHTHALMIC EMULSIONS CONTAINING PROSTAGLANDINS**

PROSTAGLANDINE ENTHALTENDE OPHTHALMISCHE EMULSIONEN

EMULSIONS OPHTALMIQUES CONTENANT DES PROSTAGLANDINES

(84) Designated Contracting States:
**AT BE BG CH CY CZ DE DK EE ES FI FR GB GR HU IE IS IT LI LT LU LV MC NL PL PT RO SE SI SK TR**

(30) Priority: **10.10.2005  EP 05011648**

(43) Date of publication of application:
**02.07.2008  Bulletin 2008/27**

(73) Proprietor: **Novagali Pharma S.A.**
**91000 Evry (FR)**

(72) Inventors:
• **PHILIPS, Betty**
**F-92160 Antony (FR)**
• **BAGUE, Séverine**
**F-91360 Epinay-sur Orge (FR)**

• **RABINOVICH-GUILATT, Laura**
**60920 Kadima (IL)**
• **LAMBERT, Grégory**
**F-92290 Chatenay Malabry (FR)**

(74) Representative: **Icosa**
**142, rue de Rennes**
**75006 Paris (FR)**

(56) References cited:
**EP-A- 0 423 697        WO-A-03/053405**
**WO-A-2005/044276    US-A- 4 684 633**
**US-A- 6 011 062**

• **DATABASE WPI Section Ch, Week 200377 Derwent Publications Ltd., London, GB; Class A96, AN 2003-826210 XP002397839 & KR 2003 046 553 A (KIM C K) 18 June 2003 (2003-06-18)**

**Description**

The present invention concerns ophthalmic cationic oil-in-water type emulsions containing prostaglandins.

In the present invention, the term « prostaglandin » is indifferently used for prostaglandin, its precursors or analogs.

The present invention is of particular interest for prostaglandin $F_{2\alpha}$ analogs such as in particular latanoprost, unoprostone isopropyl, travoprost, bimatoprost, tafluprost, 8-isoprostaglandin $E_2$ or a mixture of two or more thereof.

By « ophthalmic » it is meant an emulsion intended to be administered to the eye and which presents a pharmaceutical effect; preferably, it is topically applied.

It is known to use prostaglandins in ophthalmic preparations in order to treat glaucoma. The problem encountered with prostaglandins, in particular with latanoprost, is that their concentration lowers in the formulation overtime.

US6,011,062, US5, 688, 819, US5,849,792, US4, 599, 353 describe the use of several prostaglandin analogs for treating glaucoma and ocular hypertension. US5,849,792 discloses the use of a non ionic surfactant (polyethoxylated castor oil) to enhance the prostaglandin's chemical stability.

However, the proposed solutions to enhance the stability of prostaglandins are not completely satisfactory. Furthermore, use of BAK or other quaternary ammonium as preservative agent for prostaglandins in ophthalmic preparations has been challenged, since C. Debbasch et al. in Investigative Ophthalmology & Visual Science, March 2001, Vol42 n°3, demonstrated important toxicity of long term use of BAK and/or other quaternary ammoniums.

Latanoprost, Travoprost, Bimatoprost, unoprostone isopropyl, tafluprost, 8-isoprostaglandinE2, like most of the prostaglandin analogs, are almost insoluble in water. So, it is interesting to provide ophthalmic vehicles suitable for delivering hydrophobic drugs. In recent years, oil-in-water type emulsions, in particular emulsions having droplets of a submicron size (hereinafter "submicron emulsions") gained increasing importance. These emulsions are in general anionic emulsions. The major hurdle in developing topically applied ophthalmic drug delivery systems such as emulsions is the relatively low bioavailability of the drugs. To address this issue, cationic emulsions have been developed as topical ophthalmic vehicles; they have the advantage of increasing the bioavailability of the drugs by electrostatic attraction between the emulsions's positive charge and the negatives charges carried at the eye surface. However, stabilizing emulsions, including submicron emulsions, may be a concern for one skilled in the art. One known approach to stabilize an emulsion is to confer an electrostatic charge to the droplets surface which will result in droplet repulsion and less droplet coalescence. Colloidal particles dispersed in a solution are electrically charged due to their ionic characteristics and dipole attributes. This charge, which can be negative resulting in anionic emulsions or positive producing cationic emulsions (Klang et al., Pharm. Dev. Technology 2000, 5, 521-532) is known in the art as the "zeta potential". The zeta potential is a measure of the magnitude of the repulsion or attraction between particles (Washington, Adv. Drug Deliv. Reviews 1996, 20:131-195).

Of particular interest are the following patents dealing with cationic emulsions for topical ocular administration:

US Patent 6,007,826 discloses a cationic oil-in-water emulsion which comprises colloid particles with a positively charged interfacial film. The interfacial film is formed by cationic lipids (0.05-3% by weight) such as C10-C14 primary alkylamines (disclosed are stearylamine or oleylamine), $C_{10}$-$C_{24}$ primary alkanolamine or a cholesterol betainate; phospholipids (0.5-3%) and non-ionic surfactants from the group consisting of poloxamers, tyloxapol, polysorbate, and polyoxyethylene fatty acid esters (0.05-3%). The concentration of the oily core is maintained within the 3-20% range.

US Patent 6,007,826 emulsions zeta potential are not stable to thermal stress (see Tamilvanan et al., STP Pharma Sciences 2001, 11:421-426 and Example 12).

WO2005/044276 relates to an ophthalmic oil-in-water emulsion comprising a prostaglandine F2$\alpha$ derivative, an oil, a water-soluble polymer and water. This patent application focuses on the technical issue of the degradation of the prostaglandine and calculates latanoprost remaining rates after 1, 2, 3 or 4 weeks. However, this patent application does not show if the emulsion itself is stable overtime, i.e. for a duration exceeding 1 year.

Thus, there is still a need in ophthalmic prostaglandin products which are at least as efficient as the commercial products, which present an enhanced chemical stability of the prostaglandin, which are less toxic, which are more physically and chemically stable than conventional products, i.e. which are stable overtime and which present a good tolerability for the patient.

By overtime in the meaning of this invention, it is meant a duration exceeding 1 year, preferably exceeding 2 years, more preferably exceeding 3 years.

By "good tolerability" in the present the invention, it is understood that the ratio therapeutic benefit to ocular discomfort is acceptable by the patient, and preferably similar to a placebo or NaCl 0.9% solution.

It is generally accepted that in order to show good ocular tolerability the cation content within the formulation should not exceed 0.1%, preferably not exceed 0.05% and even more preferably should not exceed 0.03%. Quaternary amines such as benzalkonium chloride, benzododecinium bromide and benzethonium chloride are allowed by health authorities for ophthalmic administration up to concentration of approximately 0.03% (Furrer et al., Eur. J. Pharm. Biopharm. 2002, 53:263-280).

This invention thus relates to a cationic ophthalmic oil-in-water type emulsion, which comprises colloid particles having an oily core surrounded by an interfacial film,
said emulsion comprising at least one cationic agent and at least one non ionic surfactant selected from the group consisting of poloxamers, tyloxapol, polysorbates, polyoxyethylene castor oil derivatives, sorbitan esters, polyoxyl stearates and a mixture of two or more thereof, said oily core comprising a drug selected from the group consisting of latanoprost, unoprostone isopropyl, travoprost, bimatoprost, tafluprost, 8-isoprostaglandin $E_2$ or a mixture of two or more thereof, and said emulsion being free of water-soluble polymer selected from a polyvinyl compound, a water-soluble cellulose compound and a polysaccharide.

In a prefered embodiment the emulsions of the invention include latanoprost, as only drug or in combination with one or more prostaglandin selected from the group consisting of unoprostone isopropyl, travoprost, bimatoprost, tafluprost and 8-isoprostaglandin $E_2$.

According to a preferred embodiment, the emulsion of the invention is free of water-soluble polymer selected from (1) a polyvinyl compound such as polyvinylalcohol and polyvinylpyrrolidone, (2) a water-soluble cellulose compound such as methylcellulose, hydroxyethylcellulose, hydroxypropylmethylcellulose, and/or sodium cellulose and (3) a polysaccharide selected from alginic acid, xanthan gum, carrageenan, and chitosan.

According to an embodiment, the emulsion includes polyoxyethylene castor oil derivatives, especially polyethoxylated castor oil such as for example PEG-30 PEG-35 PEG-50 castor oils

According to another embodiment, the emulsion is free of any polyoxyethylene castor oil derivatives.

The term "free of" in combination with a compound or a list of compound, means that the emulsion does not contain any compound of that kind.

According to a specific embodiment of the invention, the emulsion may further comprise an anti-inflammatory compound, preferably a non steroidal anti-inflammatory compound or a omega-3 fatty acid. Anti-inflammatory agents may be chosen in the group comprising COX-2 inhibitors, salicylates, 2-arylpropionic acids, N-arylanthranilic acids, oxicams, sulphonanilides, pyrazolidines derivatives, arylalkanoic acids, 3-benzolphenylacetic acids and derivatives; steroids such as cortisone, hydrocortisone, prednisone, prednisolone, methylprednisone, fluoromethalone, medrysone, betamethasone, loteprednol, flumethasone, mometasone, testosterone, methyltestosterone, danazol, beclomethasone, dexamethasone, dexamethasone palmitate, triamcinolone, triamcinolone acetonide, fluocinolone, fluocinolone acetonide, difluprednate, rimexolone.

In the emulsions of this invention, the chemical stability of prostaglandins is enhanced. Without being linked by any theory, it is believed that since the prostaglandin is solubilized in the oily core of the emulsion, it is less available to contact with agents enhancing its degradation. Said stability is defined as the extent to which a product retains, within specified limits and throughout its period of storage and use (i.e., its shelf life), the same properties and characteristics that it possessed at the time of manufacture. The purpose of stability testing is to provide evidence on how the quality of a drug substance or drug product varies overtime under the influence of a variety of environmental factors such as temperature, humidity and light, and enables recommended storage conditions, re-test periods and shelf lives to be established.

Although real-time stability studies include an evaluation of those factors that ultimately affect the expiration date of the drugs, they are time and cost-consuming. Conventionally, accelerated stability studies are used for predicting the shelf life of pharmaceutical products. Such accelerated studies subject the systems to a temperature of 40°C during 6 months.

In order to understand the intrinsic stability mechanism of the molecule by establishing degradation pathways and identifying the likely degradation products, and thus to adjust the analytical procedures to be used, the Applicant has developed stress stability testing during which the emulsions are subjected to extreme conditions that is a temperature of 80°C during specified period of time.

The amount of prostaglandins present in the oily core of the emulsion according to the invention depends on the nature of the prostaglandins and to the intended use. According to an embodiment, the amount of the drug(s) selected from the group comprising or consisting of latanoprost, unoprostone isopropyl, travoprost, bimatoprost, tafluprost, 8-isoprostaglandin $E_2$ or a mixture of two or more thereof is of 0.001% to 1% w/w, preferably of 0.002% to 0.3% w/w and even more preferably of 0.004% to 0.15% w/w.

In the present application, percentages are expressed in weight relative to the total weight of the emulsion (% w/w).

The concentration of the cationic agent is comprised between 0.001 to 0.1% w/w, preferably between 0.002 to 0.05% w/w and even more preferably between 0.003 to 0.03%w/w.

The concentration of the oily core is not higher than 7% w/w, preferably between 0.5 to 5% w/w and even more preferably between 1 to 3%w/w.

The concentration of the non-ionic agent is less than 1% w/w, comprised preferably between 0.01 to 0.6% w/w. The cationic agent is selected in the group comprising or consisting of C10-C24 primary alkylamines, tertiary aliphatic amines, quaternary ammonium compounds selected from the group comprising benzalkonium halide, lauralkonium halide, cetrimide, hexadecyltrimethylammonium halide, tetradecyltrimethylammonium halide, dodecyltrimethylammonium halide, cetrimonium halide, benzethonium halide, behenalkonium halide, cetalkonium halide, cetethyldimonium halide, cetylpyridinium halide, benzododecinium halide, chlorallyl methenamine halide, myristalkonium halide, stearalkonium halide or a mixture of two or more thereof, halide being preferably chloride or bromide, cationic lipids, amino alcohols, biguanide salts selected from the group comprising or consisting of chlorhexidine and salts thereof, polyaminopropyl biguanide, phenformin, alkylbiguanide or a mixture of two or more thereof, cationic compounds selected from 1,2-dioleyl-3- trimethylammonium-propane, 1, 2-dioleoyl-sn-glycero- phosphatidylethanolamine, cationic glycosphingolipids or cationic cholesterol derivatives, or mixtures of two or more thereof.

According to a specific embodiment of the invention, the emulsion does not contain chlorhexidine and salts thereof. According to a preferred embodiment, the cationic agent is selected from the group comprising benzalkonium chloride, lauralkonium chloride, benzododecinium bromide, benzethenium chloride, hexadecyltrimethylammonium bromide, tetradecyltrimethylammonium bromide, dodecyltrimethylammonium bromide or a mixture of two or more thereof. According to a most preferred embodiment, the cationic agent included in the emulsion of the invention is selected from at least one quaternary ammonium halide in which the nitrogen atom is substituted by at least one alkyl group having at least 14 carbon atoms. According to a specific embodiment of the invention, the emulsion contains benzalkonium halide as only cationic agent.

The oily phase of the emulsion may comprise one or more components selected from the group comprising or consisting of vegetable oils (i.e. soybean oil, olive oil, sesame oil, cotton seed oil, castor oil, sweet almond oil), mineral oil (i.e. petrolatum and liquid paraffin), medium chain triglycerides (MCT) (i.e. a triglyceride oil in which the carbohydrate chain has about 8-12 carbon atoms), oily fatty acid, isopropyl myristate, oily fatty alcohols, esters of sorbitol and fatty acids, oily sucrose esters, and in general any oily substance which is physiologically tolerated.

According to an embodiment of the invention, the oil phase has an iodine value of less than 50, preferably equal or less than 15, more preferably equal or less than 5 and even more preferably equal or less than 1. In this embodiment, the oily phase comprises one or more components selected from the group comprising or consisting of mineral oil such as for example petrolatum and liquid paraffin, and light mineral oil, medium chain triglycerides (MCT) which is generally defined as a triglyceride oil in which the carbohydrate chain has about 8-12 carbon atoms, coconut oil; hydrogenated oils comprising hydrogenated cottonseed oil, hydrogenated palm oil, hydrogenate castor oil or hydrogenated soybean oil; polyoxyethylene hydrogenated castor oil derivatives comprising polyoxyl-40 hydrogenated castor oil, polyoxyl-60 hydrogenated castor oil or polyoxyl-100 hydrogenated castor oil.

According to a preferred embodiment of the invention, the major component of the oily phase will be either vegetable oil, preferably with an iodine value of less than 50, and/or MCT. Fatty acids or fatty alcohols may be included in cases where the hydrophobic substance to be carried by the emulsion is not sufficiently soluble in the oily phase.

Examples of MCT oil which may be used in emulsions of the present invention are TCM™ (Société des Oléagineux, France), Miglyol 812™ (Dynamit Nobel, Sweden).

The non-ionic surfactant is selected from the group comprising or consisting of poloxamers, tyloxapol, polysorbates, polyoxyethylene castor oil derivatives, sorbitan esters, polyoxyl stearates and a mixture of two or more thereof.

According to another preferred embodiment of the invention, the cationic ophthalmic emulsion comprises benzalkonium chloride as cationic agent and tyloxapol as non-ionic surfactant.

According to still another preferred embodiment, the emulsion contains benzalkonium chloride as cationic agent and a combination of tyloxapol and poloxamer as non-ionic surfactants.

The emulsion may also contain antioxidant such as Vitamin E, isotonic agent, buffering agent, preservative, etc.

According to an embodiment of the invention, the emulsion includes at least one pres-ervative. According to another embodiment of the invention, the emulsion does not contain any preservative.

The colloidal particles of the emulsions according to the invention have an average particle size of equal or less than 1 μm, advantageously equal or less than 300 nm, more advantageously in the range of 100 to 250 nm.

The emulsion of the invention may be conditionned in monodosis containers or in multidosis containers.

According to an embodiment, the containers for carrying the emulsion of the invention are made of glass, plastic materials, resins or the like.

According to another embodiment of the invention, the cationic ophthalmic emulsion may comprise a further pharmaceutically active substance, either in the oily core or in the aqueous part of the emulsion. This pharmaceutically active substance may be an antiglaucomateous active substance, which may be selected from the group comprising beta-blockers such as levobunolol, befundol, metipranolol, forskolin, carteolol, timolol; inhibitors of carbonic anhydrase such as brinzolamide, dorzolamide, acetazolamide, methazolamide, dichlorophenamide; sympathomimetics such as brimonidine, apraclonidine, dipivefrine, epinephrine; parasympathomimetics such as pilocarpine; cholinesterase inhib-

itors such as physostigmine, echothiophate and/or their derivatives; and/or optically acceptable salts thereof.

The emulsions according to the invention are physically stable overtime as defined hereabove and keep a positive zeta potential in the specific measurement conditions as described in Tests A, B, C and/or D. According to the invention, the emulsions do not contain a sufficient amount of any substances susceptible of affecting the zeta potential overtime. Advantageously, the emulsions of the invention do not contain phospholipids.

Substances susceptible of affecting the zeta potential may be phospholipids, and any substances which become negatively charged upon storage.

The amount of substances affecting the zeta potential overtime must be such that at any time, the amount of positive charge is above the amount of negative charges.

## Zeta potential

Zeta potential measures a physical property which is exhibited by any particle in suspension. Zeta potential can be used to predict behaviour of the suspension in different environments, to optimize the formulations of suspensions and emulsions as well as to predict overtime stability.

In order to avoid the emulsion droplets to adhere to one another and form aggregates of successively increasing size, it is necessary to confer repulsive forces to the particles. One of the means to confer repulsive forces to a colloidal system is by electrostatic or charge stabilization. Electrostatic or charge stabilization has the benefits of stabilizing a system by simply altering the concentration of ions in the system. This is a reversible and inexpensive process.

There might be many origins of this surface charge depending upon the nature of the particle and its surrounding medium but the most important mechanisms are the ionisation of surface groups or the adsorption of charged ions.

The interaction of particles in polar liquids is not governed by the electrical potential at the surface of the particle, but by the effective potential of the particle and its associated ions. To utilize electrostatic control of dispersions, it is the zeta potential of the particle that must be measured rather than its surface charge. Charged particles will attract ions of opposite charge in the dispersant. Ions close to the surface are strongly bound; those further away form a more diffuse region. Within this region is a notional boundary, known as the slipping plane, within which the particle and ions act as a single entity.

The potential at the slipping plane is known as the zeta potential. It has long been recognised that the zeta potential is a very good index of the magnitude of the interaction between colloidal particles and measurements of zeta potential are commonly used to assess the stability of colloidal systems. The zeta potential measured in a particular system is dependent on the chemistry of the surface, and also on the way it interacts with its surrounding environment. Therefore zeta potential must always be studied in a well defined environment (specifically pH and ionic strength).

## Electrophoretic mobility

An important consequence of the existence of electrical charges on the surface of particles is that they interact with an applied electric field. These effects are collectively defined as electrokinetic effects. If the motion is induced in a particle suspended in a liquid under the influence of an applied electric field, it is more specifically named electrophoresis. When an electric field is applied across an electrolyte, charged particles suspended in the electrolyte are attracted towards the electrode of opposite charge. Viscous forces acting on the particles tend to oppose this movement. When equilibrium is reached between these two opposing forces, the particles move with constant velocity. The velocity is dependent on the strength of electric field or voltage gradient, the dielectric constant of the medium, the viscosity of the medium and the zeta potential. The velocity of a particle in a unit electric field is referred to as its electrophoretic mobility. Zeta potential is related to the electrophoretic mobility by the Henry equation:

$$U_E = \frac{2\ \varepsilon\ z\ f(\kappa a)}{3\eta}$$

where $U_E$ = electrophoretic mobility, $z$= zeta potential, $\varepsilon$ = dielectric constant, $\eta$= viscosity and $f(\kappa a)$=Henry's function.

Electrophoretic determinations of zeta potential are most commonly made in aqueous media and moderate electrolyte concentration. $f(\kappa a)$ in this case is 1.5, and this is referred to as the Smoluchowski approximation. Therefore calculation of zeta potential from the mobility is straightforward for systems that fit the Smoluchowski model, i.e. particles larger than about 0.2 microns dispersed in electrolytes containing more that 10-3 molar salt. For small particles in low dielectric constant media (eg non-aqueous media), $f(\kappa a)$ becomes 1.0 and allows an equally simple calculation. This is referred to as the Huckel approximation.

Tests A, B, C and D

Test A consists in measuring the stability of the emulsion zeta potential under thermal stress conditions.

Zeta potential of the emulsion is measured at T=0, i.e. as soon as the emulsion has been prepared, the obtained value being named $Z_0$. Glass vials (Type I) of 10ml effective capacity containing between 5-10ml of emulsion and sealed under nitrogen atmosphere (without bubbling) are stored at 80°C.

Then at T=15 hours the zeta potential $Z_{15h}$ is measured.

The value $\delta A = Z_{15h} - Z_0$ is then calculated.

For each measurement of the zeta potential, it is operated as follows:

The zeta potential of the emulsion droplet surface is determined by electrophoretic mobility in an apparatus such as a Malvern Zetasizer 2000 (Malvern Instruments, UK) equipped with suitable software and calibrated with the supplied standard.

The emulsion is diluted in double distilled water if needed in order to obtain the scattering intensity allowing optimal particle detection. The sample count rate should be between 100 to 1000 KCps, in homodyne detection (if heterodyne detection is used, the contribution of the reference beam should be deduced). Three consecutive measurements are performed at 25°C using a constant cell drive of 150mV. The electrophoretic mobility is converted into zeta potential values through the Smoluchowsky equation, using the dielectric constants and viscosity of water. The measured value corresponds to the average of the 3 obtained values.

It is considered that the emulsion meets zeta potential stability Test A if $\delta A$ is less than the standard error of measurements, preferably less than 10mV, and even more preferably less than 5mV.

According to an advantageous embodiment, the ophthalmic emulsion according to the invention meets zeta potential stability Test B.

Test B is similar to Test A except that the emulsion is stored during 48 hours at 80°C, the zeta potential $Z_2$ is measured on day 2 and $\delta B = Z_2 - Z_0$ is calculated. The emulsion is considered as meeting the requirements of zeta potential stability test B if $\delta B$ is less than the standard error of measurements, preferably less than 10mV, and even more preferably less than 5mV.

According to a more advantageous embodiment of the invention, the ophthalmic emulsion according to the invention meets zeta potential stability Test C.

Test C is similar to Test A except that the emulsion is stored during 7 days at 80°C, the zeta potential $Z_7$ is measured on day 7 and $\delta C = Z_7 - Z_0$ is calculated. The emulsion is considered as meeting the requirements of zeta potential stability test C if $\delta C$ is less than the standard error of measurements, preferably less than 10mV, and even more preferably less than 5mV.

According to a still more advantageous embodiment of the invention, the ophthalmic emulsion according to the invention meets zeta potential stability Test D.

Test D is similar to Test A except that the emulsion is stored during 14 days at 80°C, the zeta potential $Z_{14}$ is measured on day 14 and $\delta D - Z_{14} - Z_0$ is calculated. The emulsion is considered as meeting the requirements of zeta potential stability test D if $\delta D$ is less than the standard error of measurements, preferably less than 10mV, and even more preferably less than 5mV.

According to another aspect, the invention relates to a process for manufacturing the emulsions here-above described.

The emulsions are prepared as follows:

- the prostaglandin is dissolved into the oily phase, which is optionally added with another hydrophobic ophthalmologically active ingredient,
- the aqueous phase , optionally added with another hydrophilic ophthalmologically active ingredient, is rapidly added to the oily phase,
- the coarse emulsion obtained is rapidly heated, preferably at 75°C,
- the emulsion droplet size is then decreased by any suitable means known by one skilled in the art, for example by shear mixing,
- the emulsion temperature is cooled down to 20°C using an ice bath and then homogenized
- pH is adjusted to 7-8,
- the emulsion is sterilized.

The inventions also relates to the use of a cationic ophthalmic oil-in-water emulsion as hereabove described for the preparation of an ophthalmic composition for treating ocular hypertension and/or for treating glaucoma.

According to another aspect, the invention relates to ophthalmic formulation comprising an emulsion as previously described, optionally in combination with an ophthalmologically acceptable carrier, in the form of eye drops, eye ointment, ophthalmic gel. In said ophthalmic formulation there may be a pharmaceutically effective amount of an active ingredient in or within the ophthalmologically acceptable carrier.

The invention is also directed to a delivery device selected from the group comprising lenses, ocular patch, implant, insert, said device containing an emulsion as previously described.

The invention is further illustrated by the examples below.

## EXAMPLES

In the following examples, the following abbreviations are used:

Medium Chain Triglycerides MCT: TCM™ (Société des Oléagineux)
BAK: benzalkonium chloride (FeF Chemicals, Denmark)
Lutrol: Lutrol F68™ (BASF)
Tyloxapol : Triton WR1339 (Ruger Chemical USA)
Z29 : latanoprost

## Example 1

| Emulsion | Z29EM002 | Z29EM003 | Z29EM005 | Z29EM007 |
|---|---|---|---|---|
| Composition | 1% MCT<br>0.1 % Lipid<br>0.05% OA<br>0.005% vit E<br>0.25% Lutrol<br>2.25% Glycerin<br>Water to 100%<br>Z29 0.005% | 1% MCT<br>0.1% Tyloxapol<br>0.05% OA<br>0.005% vit E<br>0.25% Lutrol<br>2.25% Glycerin<br>Water to 100%<br>Z29 0.005% | 0.02% BAK<br>1 % MCT<br>0.16% Tyloxapol<br>0.01% vit E<br>0.25% Lutrol<br>2.25% Glycerin<br>Water to 100%<br>Z29 0.005% | 0.02% BAK<br>1% MCT<br>0.3% Tyloxapol<br>0.01% vit E<br>0.1% Lutrol<br>2.25% Glycerin<br>Water to 100%<br>Z29 0.005% |
| Zeta potential stress test | | | **T0**: 22:4<br>**T7**: 24.1<br>**T15**: 19.8 | **T0**: 21.8<br>**T7**: 18.8<br>**T15**: 18.9 |
| Droplet size (nm) stress test | | | **T0**: 160<br>**T7**: 173<br>**T15**: 185 | **T0**: 212<br>**T7**: 225<br>**T15**: 236 |

| Emulsion | Z29EM008 | Z29EM011 |
|---|---|---|
| Composition | 0.02% BAK<br>1% MCT<br>0.3% Tyloxapol<br>0.1% Lutrol<br>2.25% Glycerin<br>Water to 100%<br>Z29 0.005% | 0.01% BAK<br>1% MCT<br>0.3% Tyloxapol<br>0.1% Lutrol<br>2.25% Glycerin<br>Water to 100%<br>Z29 0.005% |
| Zeta potential stress test | **T0**: 20.6<br>**T7**: 18.5<br>**T15**: 16.2 | |
| Droplet size stress test | **T0**: 201<br>**T7**: 212<br>**T15**: 216 | |

The oily phase components including 0.005% latanoprost (named Z29 in the Tables) were successively weighed in the same beaker and then magnetically stirred under a slight heating (40°C) until a slightly viscous phase is obtained. Aqueous phase components were successively weighed in the same beaker and then magnetically stirred under a slight heating (40°C) until a transparent, limpid and fluid phase is obtained. Both phases were heated to 65°C. The coarse emulsion was formed by rapid addition of the aqueous phase in the oily phase and was then rapidly heated to 75°C. The aqueous phase and coarse emulsion beakers were protected by a film to avoid any water evaporation. The emulsion was white and slightly transparent. The emulsion droplet size was then decreased by a 5 minutes high shear mixing with a POLYTRON PT 6100. The emulsion became milky. The emulsion temperature was cooled down to 20°C using an ice batch.

The final emulsion was obtained by homogenization in a microfluidizer (C5, Avestin) using continuous cycles for 5 min at a pressure of 10,000 psi. The emulsion was milky, very fluid and did not adhere on the glass. The emulsion temperature was decreased to 25°C. Its pH was measured and then adjusted to 7.0 using a 0.1 M HCl or 0.1 M NaOH solution. Emulsion was conditioned in glass vials with nitrogen bubbling and then sterilized in an autoclave 20 minutes at 121°C.

The mean particle size of the emulsions droplets was determined by quasi-elastic light scattering after dilution in water using a High Performance Particle Sizer (Malvern Instruments, UK).

The electrophoretic mobility was measured at 25°C in a Malvern Zetasizer 2000 (Malvern Instruments, UK) following a 1:200 dilution in double dis-tilled water as detailed above and converted into zeta potential through the Smoluchowski equation.

## Example 2

Latanoprost stability improvement in emulsion compared to commercial product (Xalatan®)

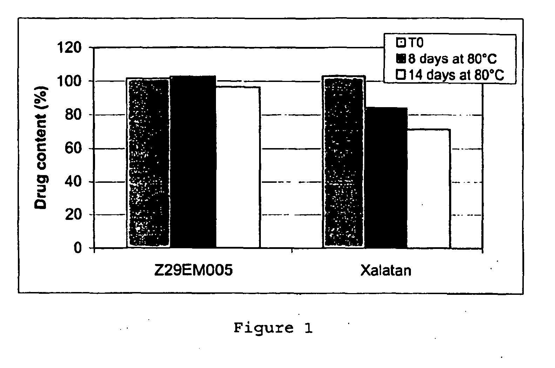

The chemical stability of latanoprost within the emulsion was compared to the commercial product Xalatan® at 80°C for 14 days (figure 1).

Prostaglandin contents were analysed by an HPLC-W method.

In emulsions according to the invention, latanoprost is chemically stabilized.

## Example 3:

In vivo studies demonstrating that latanoprost emulsion is as efficient as the commercial product (Xalatan®) in reducing IOP (intraocular pressure)

Methods: Eight adult female cynomolgus monkeys, each weighing 3-6 kg, in which glaucoma had been induced by diode laser photocoagulation of the mid-trabecular meshwork, were used in this study. Intraocular pressure (IOP) was measured at 0 hr (before drug administration) and then hourly until 6 hrs after drug administration for one baseline day, one vehicle-treated day, and treatment days 1,3, and 5 with 30μl of Z29EM007 (similar to the emulsion described in Example 1) or 0.005% Latanoprost (Xalatan; Pharmarcia & Upjohn, Kalamazoo, MI).

The products were topically applied to the glaucomatous eye once daily for 5 consecutive days in a crossover design with a washout period at least 2 weeks between the two drugs.

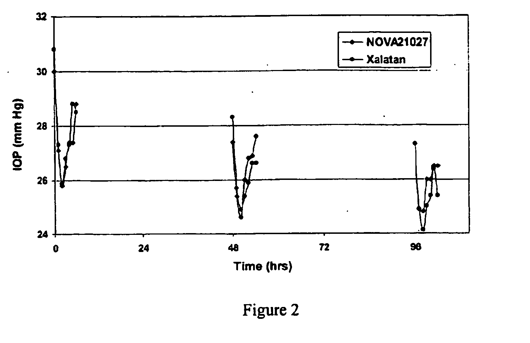

Results: Once daily administration of both Z29EM007 and Xalatan for 5 days significantly ($p < 0.005$) reduced IOP from 1 hr to 5 hrs after the first dose compared to the vehicle treatment day (figure 2).

The ocular hypotensive effect was enhanced by repeated dosing for both Z29EM007 and Xalatan. No statistical difference of IOP reduction ($p > 0.80$) was observed during the 5 days treatment when comparing Z29EM007 and Xalatan. IOP on the baseline day and vehicle-treated day was not statistically different between the two drugs ($p > 0.90$).

Latanoprost in the emulsions according to the invention is as efficient as commercially available Xalatan™.

## Claims

**1.** A cationic ophthalmic oil-in-water type emulsion, which comprises colloid particles having an oily core surrounded by an interfacial film,
said emulsion comprising at least one cationic agent and at least one non ionic surfactant selected from the group consisting of poloxamers, tyloxapol, polysorbates, polyoxyethylene castor oil derivatives, sorbitan esters, polyoxyl stearates and a mixture of two or more thereof, said oily core comprising a drug selected from the group consisting of latanoprost, unoprostone isopropyl, travoprost, bimatoprost, tafluprost, 8-isoprostaglandin E2 or a mixture of two or more thereof
said emulsion being free of water-soluble polymer selected from a polyvinyl compound, a water-soluble cellulose compound and a polysaccharide.

**2.** A cationic ophthalmic oil-in-water type emulsion according to claim **1,** wherein the drug is latanoprost.

**3.** A cationic ophthalmic oil-in-water type emulsion according to any of claims **1** or **2,** said emulsion being free of water-soluble polymer selected from (1) a polyvinyl compound such as polyvinylalcohol and polyvinylpyrrolidone, (2) a water-soluble cellulose compound such as methylcellulose, hydroxyethylcellulose, hydroxypropylmethylcellulose, and/or sodium cellulose and (3) a polysaccharide selected from alginic acid, xanthan gum, carrageenan, and chitosan.

**4.** A cationic ophthalmic emulsion according to anyone of claims **1** to **3,** comprising at least one further pharmaceutically active substance, either in the oily core or in the aqueous part of the emulsion.

**5.** A cationic ophthalmic oil-in-water type emulsion according to any of claims **1** to **4,** further comprising an anti-inflammatory compound, preferably a non steroidal anti-inflammatory compound or an omega-3 fatty acid.

**6.** A cationic ophthalmic oil-in-water type emulsion according to any of claims **1** to **4,** wherein the emulsion further comprises at least one further anti-glaucomateous pharmaceutically active substance selected from the group comprising beta-blockers such as levobunolol, befundol, forskolin, metipranolol, carteolol, timolol; inhibitors of carbonic anhydrase such as brinzolamide, dorzolamide, acetazolamide, methazolamide, dichlorophenamide; sympathomimetics such as brimonidine, apraclonidine, dipivefrine, epinephrine; parasympathomimetics such as pilocarpine; cholinesterase inhibitors such as physostigmine, echothiophate and/or their derivatives; and/or optically acceptable salts thereof.

**7.** A cationic ophthalmic oil-in-water type emulsion according to anyone of claims **1** to **6,** wherein the amount of the drug selected from the group consisting of latanoprost, unoprostone isopropyl, travoprost, bimatoprost, tafluprost, 8-isoprostaglandin E2 or a mixture of two or more thereof in the oily core is 0.001 to 1% w/w, preferably 0.002 to 0.3% w/w and even more preferably 0.004 to 0.15% w/w.

**8.** A cationic ophthalmic oil-in-water type emulsion according to anyone of claims **1** to **7,** wherein the concentration of the cationic agent is comprised between 0.001 to 0.1% w/w, preferably between 0.002 to 0.05% w/w and even more preferably between 0.003 to 0.03% w/w.

**9.** A cationic ophthalmic oil-in-water type emulsion according to anyone of claims **1** to **8,** wherein the concentration of the oily core is not higher than 7% w/w, preferably between 0.5 to 5% w/w and even more preferably between 1 to 3% w/w.

**10.** A cationic ophthalmic emulsion according to anyone of claims **1** to **9,** wherein the concentration of the non-ionic agent is less than 1% w/w, comprised preferably from 0.01 to 0.6% w/w.

**11.** A cationic ophthalmic oil-in-water emulsion according to anyone of claims **1** to **10,** wherein the cationic agent is selected in the group consisting of C10-C24 primary alkylamines, tertiary aliphatic amines, quaternary ammonium compounds, cationic lipids, amino alcohols, biguanide salts, cationic compounds and a mixture of two or more thereof.

**12.** A cationic ophthalmic oil-in-water emulsion according to claim **11,** wherein the biguanide salt is selected from the group comprising chlorhexidine and salts thereof, polyaminopropyl biguanide, phenformin, alkylbiguanide or a mixture of two or more thereof.

**13.** A cationic ophthalmic oil-in-water emulsion according to claim **11,** wherein the quaternary ammonium compound is selected from the group comprising benzalkonium halide, lauralkonium halide, cetrimide, hexadecyltrimethylammonium halide, tetradecyltrimethylammonium halide, dodecyltrimethylammonium halide, cetrimonium halide, benzethonium halide, behenalkonium halide, cetalkonium halide, cetethyldimonium halide, cetylpyridinium halide, benzododecinium halide, chlorallyl methenamine halide, myristylalkonium halide, stearalkonium halide or a mixture of two or more thereof, halide being preferably chloride or bromide.

**14.** A cationic ophthalmic emulsion according to anyone of claims **1** to **10,** wherein said cationic agent is selected from the group comprising benzalkonium chloride, lauralkonium chloride, benzododecinium bromide, benzethonium chloride, hexadecyltrimethylammonium bromide, tetradecyltrimethylammonium bromide, dodecyltrimethylammonium bromide or a mixture of two or more thereof.

15. A cationic ophthalmic emulsion according to anyone of claims **1** to **10,** wherein the cationic agent is selected from at least one quaternary ammonium halide in which the nitrogen atom is substituted by at least one alkyl group having at least 14 carbon atoms.

16. A cationic ophthalmic emulsion according to anyone of claims **1** to **15,** wherein the oily phase has a iodine value of less than 50, preferably equal or less than 15, more preferably equal or less than 5 and even more preferably equal or less than 1.

17. A cationic ophthalmic emulsion according to claim **16,** wherein the oily phase comprises one or more components selected from the group consisting of mineral oil and light mineral oil, medium chain triglycerides (MCT), coconut oil; hydrogenated oils comprising hydrogenated cottonseed oil, hydrogenated palm oil, hydrogenated castor oil or hydrogenated soybean oil; polyoxyethylene hydrogenated castor oil derivatives comprising polyoxyl-40 hydrogenated castor oil, polyoxyl-60 hydrogenated castor oil or polyoxyl-100 hydrogenated castor oil.

18. A cationic ophthalmic emulsion according to anyone of claims **1** to **17,** wherein the oil is MCT.

19. A cationic ophthalmic emulsion according to anyone of claims **1** to **18,** comprising benzalkonium chloride as cationic agent and tyloxapol as non-ionic surfactant.

20. A cationic ophthalmic emulsion according to anyone of claims **1** to **19,** wherein the emulsion contains benzalkonium chloride as cationic agent and a combination of tyloxapol and poloxamer.

21. A cationic ophthalmic emulsion according to anyone of claims **1** to **20,** wherein said colloidal particles have an average particle size of equal or less than 1 [mu]m, advantageously equal or less than 300 nm, more advantageously in the range of 100 to 250 nm.

22. A cationic ophthalmic oil-in-water type emulsion according to anyone of claims **1** to **21,** wherein the emulsion meets zeta potential stability Test A requirements as defined in the description, wherein the zeta potential of the emulsion is measured at T=0, the emulsion is stored at 80°C during 15 hours and measured at T=15 hours, the emulsion meeting zeta potential stability Test A requirements when the variation of zeta potential, delta A = $Z_{15h} - Z_0$, between the two measurements, is of less than the standard error of measurements, preferably less than 10mV.

23. An ophthalmic emulsion according to anyone of claims **1** to **21,** which meets zeta potential stability Test B requirements, as defined in the description, wherein the zeta potential $Z_0$ of the emulsion is measured at T=0, then the emulsion is stored during 48 hours at 80°C, and the zeta potential $Z_2$ of the emulsion is measured on day 2, the emulsion meeting zeta potential stability Test B requirements when the variation of zeta potential, delta B = $Z_2 - Z_0$, between the two measurements is of less than the standard error of measurements, preferably less than 10mV.

24. An ophthalmic emulsion according to anyone of claims **1** to **21,** which meets zeta potential stability Test C requirements, as defined in the description, wherein the zeta potential Zo of the emulsion is measured at T=0, then the emulsion is stored during seven days at 80°C, and the zeta potential $Z_7$ of the emulsion is measured on day 7, the emulsion meeting zeta potential stability Test C requirements when the variation of zeta potential, delta C = $Z_7 - Z_0$, between the two measurements is of less than the standard error of measurements, preferably less than 10mV.

25. An ophthalmic emulsion according to anyone of claims **1** to **21,** which meets zeta potential stability Test D requirements, as defined in the description, wherein the zeta potential $Z_0$ of the emulsion is measured at T=0, then the emulsion is stored during 14 days at 80°C, and the zeta potential $Z_{14}$ of the emulsion is measured on day 14, the emulsion meeting zeta potential stability Test D requirements when the variation of zeta potential, delta D = $Z_{14} - Z_0$, between the two measurements is of less than the standard error of measurements, preferably

26. An ophthalmic emulsion according to anyone of claims **1** to **25,** said emulsion being in the form of eye drops, eye ointment, ophthalmic gel.

27. An ophthalmic emulsion according to anyone of claims **1** to **25,** wherein the emulsion is within a delivery device selected from the group comprising lenses, ocular patch, implant and insert.

28. Use of a cationic ophthalmic oil-in-water emulsion according to any one of claims **1** to **27,** for the preparation of an ophthalmic composition for treating ocular hypertension and/or for treating glaucoma.

**Patentansprüche**

1. Kationische Öl-in-Wasser-artige Augenmulsion, die Kolloidpartikel mit einem öligen Kern umfasst, welche von einem zwischenflächigen Film umgeben sind, wobei die Emulsion mindestens ein kationisches Mittel und mindestens ein nicht ionisches Tensid, ausgewählt aus der Gruppe bestehend aus Poloxameren, Tyloxapol, Polysorbaten, Polyoxyethylen-Rizinusölderivaten, Sorbitanestern, Polyoxylstearaten und einer Mischung von zwei oder mehreren davon, umfasst, wobei der ölige Kern einen Wirkstoff, ausgewählt aus der Gruppe bestehend aus Latanoprost, Unoprostonisopropyl, Travoprost, Bimatoprost, Tafluprost, 8-Isoprostaglandin-E2 oder einer Mischung von zwei oder mehreren davon, umfasst, wobei die Emulsion frei von wasserlöslichem Polymer, ausgewählt aus einer Polyvinylverbindung, einer wasserlöslichen Celluloseverbindung und einem Polysacharid, ist.

2. Kationische Öl-in-Wasser-artige Augenemulsion gemäß Anspruch 1, wobei der Wirkstoff Latanoprost ist.

3. Kationische Öl-in-Wasser-artige Augenemulsion gemäß einem beliebigen der Ansprüche 1 oder 2, wobei die Emulsion frei von wasserlöslichem Polymer, ausgewählt aus (1) einer Polyvinylverbindung wie Polyvinylalkohol und Polyvinylpyrrolidon, (2) einer wasserlöslichen Celluloseverbindung wie Methylcellulose, Hydroxyethylcellulose, Hydroxypropylmethylcellulose und/oder Natriumcellulose und (3) einem Polysacharid, ausgewählt aus Alginsäure, Xanthangummi, Carrageenan und Chitosan, ist.

4. Kationische Augenemulsion gemäß einem beliebigen der Ansprüche 1 bis 3, umfassend mindestens eine weitere pharmazeutisch aktive Substanz, entweder in dem öligen Kern oder in dem wässrigen Teil der Emulsion.

5. Kationische Öl-in-Wasser-artige Augen-Emulsion gemäß einem beliebigen der Ansprüche 1 bis 4, des Weiteren umfassend eine antiinflammatorische Verbindung, vorzugsweise eine nicht steroidale antiinflammatorische Verbindung oder eine Omega-3-Fettsäure.

6. Kationische Öl-in-Wasser-artige Augen-Emulsion gemäß einem beliebigen der Ansprüche 1 bis 4, wobei die Emulsion des Weiteren mindestens eine weitere antiglaucomatöse pharmazeutisch aktive Substanz, ausgewählt aus der Gruppe bestehend aus Beta-Blockern wie Levobunolol, Befundol, Forskolin, Metipranolol, Cartrolol, Timolol; Inhibitoren von Carboanhydrase wie Brinzolamid, Dorszolamid, Acetazolamid, Methazolamid, Dichlorphenamid; Sympathomimetika wie Brimonidin, Apraclonidin, Dipivefrin, Epinephrin; Parasympathomimetika wie Pilocarpin; Cholinesteraseinhibitoren wie Physostigmin, Echothiophat und/oder deren Derivate; und/oder optisch akzeptable Salze davon umfasst.

7. Kationische Öl-in-Wasser-artige Augenemulsion gemäß einem beliebigen der Ansprüche 1 bis 6, wobei die Menge des Wirkstoffs, ausgewählt aus der Gruppe bestehend aus Latanoprost, Unoprostonisopropyl, Travoprost, Bimatoprost, Tafluprost, 8-Isoprostaglandin-E2 oder einer Mischung von zwei oder mehreren davon, in dem öligen Kern 0,001 bis 1 Gew.-%, vorzugsweise 0,002 bis 0,3 Gew.-% und noch bevorzugter 0,004 bis 0,15 Gew.-% ist.

8. Kationische Öl-in-Wasser-artige Augenemulsion gemäß einem beliebigen der Ansprüche 1 bis 7, wobei die Konzentration des kationischen Mittels zwischen 0,001 bis 0,1 Gew.-%, vorzugsweise zwischen 0,002 bis 0,05 Gew.-% und noch bevorzugter zwischen 0,003 bis 0,03 Gew.-% umfasst ist.

9. Kationische Öl-in-Wasser-artige Augen-Emulsion gemäß einem beliebigen der Ansprüche 1 bis 8, wobei die Konzentration des öligen Kerns nicht höher als 7 Gew.-%, vorzugsweise zwischen 0,5 bis 5 Gew.-% und noch bevorzugter zwischen 1 bis 3 Gew.-% ist.

10. Kationische Augenemulsion gemäß einem beliebigen der Ansprüche 1 bis 9, wobei die Konzentration des nicht ionischen Mittels weniger als 1 Gew.-%, vorzugsweise von 0,01 bis 0,6 Gew.-% umfasst ist.

11. Kationische Öl-in-Wasser-Augenemulsion gemäß einem beliebigen der Ansprüche 1 bis 10, wobei das kationische Mittel ausgewählt ist aus der Gruppe bestehend aus C10-C24 primären Alkylaminen, tertiären aliphatischen Aminen, quaternären Ammoniumverbindungen, kationischen Lipiden, Aminoalkoholen, Biguanidsalzen, kationischen Verbindungen und einer Mischung von zwei oder mehreren davon.

12. Kationische Öl-in-Wasser-artige Augen-Emulsion gemäß Anspruch 11, wobei das Biguanidsalz ausgewählt ist aus der Gruppe umfassend Chlorhexidin und Salzen davon, Polyaminopropylbiguanid, Phenformin, Alkylbiguanid oder einer Mischung von zwei oder mehreren davon.

13. Kationische Öl-in-Wasser-Augenemulsion gemäß Anspruch 11, wobei die quaternäre Ammoniumverbindung ausgewählt ist aus der Gruppe bestehend aus Benzalkoniumhalogenid, Lauralkoniumhalogenid, Cetrimid, Hexadecyltrimethylammoniumhalogenid, Tetradecyltrimethylammoniumhalogenid, Dodecyltrimethylammoniumhalogenid, Cetrimoniumhalogenid, Benzethoniumhalogenid, Behenalkoniumhalogenid, Cetalkoniumhalogenid, Cetethyldimoniumhalogenid, Cetylpyridiniumhalogenid, Benzododeciniumhalogenid, Chlorallylmethenaminhalogenid, Myristylalkoniumhalogenid, Stearylalkoniumhalogenid oder einer Mischung von zwei oder mehreren davon, wobei die Halogenide vorzugsweise Chlorid oder Bromid sind.

14. Kationische Augenemulsion gemäß einem beliebigen der Ansprüche 1 bis 10, wobei das kationische Mittel ausgewählt ist aus der Gruppe bestehend aus Benzalkoniumchlorid, Lauralkoniumchlorid, Benzododeciniumbromid, Benzetheniumchlorid, Hexadecyltrimethylammoniumbromid, Tetradecyltrimethylammoniumbromid, Dodecyltrimethylammoniumbromid oder einer Mischung von zwei oder mehreren davon.

15. Kationische Augenemulsion gemäß einem beliebigen der Ansprüche 1 bis 10, wobei das kationische Mittel ausgewählt ist aus mindestens einem quaternären Ammoniumhalogenid, in welchem das Stickstoffatom durch mindestens eine Alkylgruppe mit mindestens 14 Kohlenstoffatomen substituiert ist.

16. Kationische Augenemulsion gemäß einem beliebigen der Ansprüche 1 bis 15, wobei die Ölphase einen Iodwert von weniger als 50, vorzugsweise gleich oder weniger als 15, stärker bevorzugt gleich oder weniger als 5 und noch stärker bevorzugt gleich oder weniger als 1 hat.

17. Kationische Augenemulsion gemäß Anspruch 16, wobei die Ölphase eine oder mehrere Verbindungen, ausgewählt aus der Gruppe bestehend aus Mineralöl und leichtem Mineralöl, mittelkettigen Triglyceriden (MCT), Kokosnussöl; gehärteten Ölen umfassend gehärtetes Baumwollsamenöl, gehärtetes Palmöl, gehärtetes Rizinusöl, oder gehärtetes Sojabohnenöl; Polyoxyethylen-gehärtete Rizinusölderivate umfassend Polyoxy-40-gehärtetes Rizinusöl, Polyoxy-60-gehärtetes Rizinusöl oder Polyoxy-100-gehärtetes Rizinusöl, umfasst.

18. Kationische Augenemulsion gemäß einem beliebigen der Ansprüche 1 bis 17, wobei das Öl MCT ist.

19. Kationische Augenemulsion gemäß einem beliebigen der Ansprüche 1 bis 18, umfassend Benzalkoniumchlorid als kationisches Mittel und Tyloxapol als nicht ionisches Tensid.

20. Kationische Augenemulsion gemäß einem beliebigen der Ansprüche 1 bis 19, wobei die Emulsion Benzalkoniumchlorid als kationisches Mittel und eine Kombination von Tylopaxol und Poloxamer enthält.

21. Kationische Augenemulsion gemäß einem beliebigen der Ansprüche 1 bis 20, wobei die kolloidalen Partikel eine durchschnittliche Partikelgröße von gleich oder weniger als 1 [mu]m haben, vorteilhafterweise gleich oder weniger als 300 nm, noch vorteilhafterweise im Bereich von 100 bis 250 nm.

22. Kationische Öl-in-Wasser-artige Augenemulsion gemäß einem beliebigen der Ansprüche 1 bis 21, wobei die Emulsion Voraussetzungen des Zetapotential-Stabilitätstests A, wie in der Beschreibung definiert, erfüllt, wobei das Zetapotential der Emulsion bei T = 0 gemessen wird, die Emulsion bei 80 °C für 15 Stunden gelagert und bei T = 15 Stunden gemessen wird, die Emulsion Voraussetzungen des Zetapotential-Stabilitätstests A erfüllt, wenn die Abweichung des Zetapotentials, Delta A = $Z_{15h}$-$Z_0$, zwischen den zwei Messungen weniger als die Standardabweichung der Messungen, vorzugsweise weniger als 10 mV, beträgt.

23. Augenemulsion gemäß einem beliebigen der Ansprüche 1 bis 21, die Voraussetzungen des Zetapotential-Stabilitätstests B, wie in der Beschreibung definiert, erfüllt, wobei das Zetapotential $Z_0$ der Emulsion bei T = 0 gemessen wird, die Emulsion dann für 48 Stunden bei 80 °C gelagert wird und das Zetapotential $Z_2$ der Emulsion am Tag 2 gemessen wird, wobei die Emulsion Voraussetzungen des Zetapotential-Stabilitätstests B erfüllt, wenn die Abweichung des Zetapotentials, Delta B = $Z_2$ - $Z_0$, zwischen den zwei Messungen weniger als die Standardabweichung der Messungen, vorzugsweise weniger als 10 mV, beträgt.

24. Augenemulsion gemäß einem beliebigen der Ansprüche 1 bis 21, die Voraussetzungen des Zetapotential-Stabilitätstests C, wie in der Beschreibung definiert, erfüllt, wobei das Zetapotential $Z_0$ der Emulsion bei T = 0 gemessen wird, die Emulsion dann für 7 Tage bei 80 °C gelagert wird und das Zetapotential $Z_7$ der Emulsion am Tag 7 gemessen wird, die Emulsion Voraussetzungen des Zetapotential-Stabilitätstests erfüllt, wenn die Abweichung des Zetapotentials, Delta C = $Z_7$ - $Z_0$, zwischen den zwei Messungen weniger als die Standardabweichung der Messungen, vorzugsweise weniger als 10 mV, beträgt.

25. Augenemulsion gemäß einem beliebigen der Ansprüche 1 bis 21, die Voraussetzungen des Zetapotential-Stabilitätstests D, wie in der Beschreibung definiert, erfüllt, wobei das Zetapotential $Z_0$ der Emulsion bei T = 0 gemessen wird, die Emulsion dann für 14 Tage bei 80 °C gelagert wird und das Zetapotential $Z_{14}$ der Emulsion am Tag 14 gemessen wird, die Emulsion Voraussetzungen des Zetapotential-Stabilitätstests erfüllt, wenn die Abweichung des Zetapotentials, Delta D = $Z_{14}$ - $Z_0$, zwischen den zwei Messungen weniger als die Standardabweichungen der Messungen, vorzugsweise weniger als 10 mV, beträgt.

26. Augenemulsion gemäß einem beliebigen der Ansprüche 1 bis 25, wobei die Emulsion in Form von Augentropfen, Augensalbe, Augengel ist.

27. Augenemulsion gemäß einem beliebigen der Ansprüche 1 bis 25, wobei die Emulsion in einer Lieferungsvorrichtung ausgewählt aus der Gruppe bestehend aus Linsen, Augenpflaster, Implantat und Einsatz ist.

28. Verwendung einer kationischen Öl-in-Wasser-Augenemulsion gemäß einem beliebigen der Ansprüche 1 bis 27 für die Herstellung einer Augenzusammensetzung zum Behandeln von Augenhypertonie und/oder zum Behandeln von Glaukom.

## Revendications

1. Émulsion ophtalmique cationique type huile-dans-eau, qui comprend des particules colloïdales ayant un coeur huileux entouré d'un film interfacial,
ladite émulsion comprenant au moins un agent cationique et au moins un tensio-actif non-ionique sélectionné dans le groupe constitué des poloxamères, du tyloxapol, des polysorbates, des dérivés d'huile de ricin polyoxyéthylénés, des esters de sorbitan, des stéarates polyoxyléthyléniques et un mélange de deux ou plus de ceux-ci, ledit coeur huileux comprenant un principe actif sélectionné parmi le groupe constitué du latanoprost, de l'unoprostone isopropyle, du travoprost, du bimatoprost, du tafluprost, de la 8-isoprostaglandine E2 ou un mélange de deux ou plus de ceux-ci,
ladite émulsion étant exempte de polymère hydrosoluble sélectionné parmi les composés polyvinyles, les composés celluloses hydrosolubles and les polysaccharides.

2. Émulsion ophtalmique cationique type huile-dans-eau selon la revendication **1,** dans laquelle le principe actif est le latanoprost.

3. Émulsion ophtalmique cationique type huile-dans-eau selon l'une quelconque des revendications **1** ou **2,** ladite émulsion étant exempte d'un polymère hydrosoluble sélectionné parmi (1) un composé polyvinyle tel que l'alcool de polyvinyle et le polyvinylpyrrolidone, (2) un composé cellulose hydrolosuble tel que la methylcellulose, l'hydroxyéthylcellulose, l'hydroxypropylméthylcellulose, et/ou le cellulose sodique et (3) un polysaccharide sélectionné parmi l'acide alginique, la gomme xanthane, le carraghénane, et le chitosan.

4. Émulsion ophtalmique cationique type huile-dans-eau selon l'une quelconque des revendications **1** à **3,** comprenant au moins une autre substance pharmaceutiquement active, soit dans le coeur huileux, soit dans la partie aqueuse de l'émulsion.

5. Émulsion ophtalmique cationique type huile-dans-eau selon l'une quelconque des revendications **1** à **4,** comprenant en plus un composé anti-inflammatoire, préférablement un composé anti-inflammatoire non stéroïdien ou un acide gras oméga-3.

6. Émulsion ophtalmique cationique type huile-dans-eau selon l'une quelconque des revendications **1** à **4,** dans laquelle l'émulsion comprend également une autre substance anti-glaucomateuse pharmaceutiquement active sélectionnée dans le groupe comprenant les bêta-bloquants tels que le lévobunolol, le béfundol, le forskolin, le métipranolol, le cartéolol, le timolol; les inhibiteurs de l'anhydrase carbonique tels que la brinzolamide, la dorzolamide, l'acétazolamide, la méthazolamide, la dichlorophénamide; les sympathomimétiques tels que la brimonidine, l'apraclonidine, la dipivefrine, l'épinéphrine; les parasympathomimétiques tels que la pilocarpine; les inhibiteurs de cholinestérase tels que la physostigmine, l'échothiophate et/ou leurs dérivés; et/ou les sels ophtalmologiquement acceptables de ceux-ci.

7. Émulsion ophtalmique cationique type huile-dans-eau selon l'une quelconque des revendications **1** à **6,** dans laquelle la quantité de principe actif, sélectionné dans le groupe constitué du latanoprost, de l'unoprostone isopropyle, du travoprost, du bimatoprost, du tafluprost, de la 8-isoprostaglandine $E_2$ ou d'un mélange de deux ou plus de ceux-ci dans le coeur huileux, est comprise entre 0,001 et 1% m/m, préférablement entre 0,002 et 0,3% m/m et encore plus préférablement entre 0,004 et 0,15% m/m.

8. Émulsion ophtalmique cationique type huile-dans-eau selon l'une quelconque des revendications **1** à **7,** dans laquelle la concentration de l'agent cationique est comprise entre 0,001 et 0,1% m/m, préférablement entre 0,002 et 0,05% m/m et encore plus préférablement entre 0,003 et 0,03% m/m.

9. Émulsion ophtalmique cationique type huile-dans-eau selon l'une quelconque des revendications **1** à **8,** dans laquelle la concentration du coeur huileux n'est pas supérieure à 7% m/m, préférablement entre 0,5 et 5% m/m et encore plus préférablement entre 1 et 3%m/m.

10. Émulsion ophtalmique cationique type huile-dans-eau selon l'une quelconque des revendications **1** à **9,** dans laquelle la concentration de l'agent non-ionique est inférieure à 1% m/m, comprise de préférence entre 0,01 et 0,6% m/m.

11. Émulsion ophtalmique cationique type huile-dans-eau selon l'une quelconque des revendications **1** à **10,** dans laquelle l'agent cationique est sélectionné dans le groupe constitué des alkylamines primaires en C10-C24, des amines aliphatiques tertiaires, des composés ammoniums quarternaires, des lipides cationiques, des amino alcools, des sels de biguanide, des composés cationiques et un mélange de deux ou plus de ceux-ci.

12. Émulsion ophtalmique cationique type huile-dans-eau selon la revendication **11,** dans laquelle le sel de biguanide est sélectionné dans le groupe comprenant la chlorhexidine et ses sels, le polyaminopropyl biguanide, la phenformine, l'alkylbiguanide ou un mélange de deux ou plus de ceux-ci.

13. Émulsion ophtalmique cationique type huile-dans-eau selon la revendication **11,** dans lequel le composé ammonium quaternaire est sélectionné dans le groupe comprenant de l'halogénure de benzalkonium, de l'halogénure de lauralkonium, du cetrimide, de l'halogénure de hexadécyltriméthylammonium, de l'halogénure de tétradécyltriméthylammonium, de l'halogénure de dodécyltriméthylammonium, de l'halogénure de cétrimonium, de l'halogénure de benzéthonium, de l'halogénure de béhénalkonium, de l'halogénure de cétalkonium, de l'halogénure de cététhyldimonium, de l'halogénure de cétylpyridinium, de l'halogénure de benzododécinium, de l'halogénure de chlorallyl méthénamine, de l'halogénure de myristylalkonium, de l'halogénure de stéaralkonium ou un mélange de deux ou plus de ceux-ci, l'halogénure étant de préférence du chlorure ou du bromure.

14. Émulsion ophtalmique cationique selon l'une quelconque des revendications **1** à **10,** dans laquelle ledit agent cationique est sélectionné dans le groupe comprenant le chlorure de benzalkonium, le chlorure de lauralkonium, le bromure de benzododécinium, le chlorure de benzethonium, le bromure d'héxadécyltriméthylammonium, le bromure de tétradécyltriméthylammonium, le bromure de dodécyltriméthylammonium ou un mélange de deux ou plus de ceux-ci.

15. Émulsion ophtalmique cationique selon l'une quelconque des revendications **1** à **10,** dans laquelle l'agent cationique est sélectionné parmi au moins un halogénure d'ammonium quaternaire dans lequel l'atome d'azote est substitué par au moins un groupe alkyle ayant au moins 14 atomes de carbone.

16. Émulsion ophtalmique cationique selon l'une quelconque des revendications **1** à **15,** dans laquelle la phase huileuse a un indice d'iode de moins de 50, préférablement inférieur ou égal à 15, plus préférablement inférieur ou égal à 5 et encore plus préférablement inférieur ou égal à 1.

17. Émulsion ophtalmique cationique selon la revendication **16,** dans laquelle la phase huileuse comprend un ou plusieurs composants sélectionnés dans le groupe composé de l'huile de paraffine et de l'huile de paraffine légère, des triglycérides à chaîne moyenne (TCM), de l'huile de noix de coco; des huiles hydrogénées comprenant l'huile de coton hydrogénée, l'huile de palme hydrogénée, l'huile de ricin hydrogénée or l'huile de soja hydrogénée; les dérivés d'huile de ricin polyoxyéthylénée hydrogénée comprenant l'huile de ricin polyoxyl-40 hydrogénée, l'huile de ricin polyoxyl-60 hydrogénée ou l'huile de ricin polyoxyl-100 hydrogénée.

18. Émulsion ophtalmique cationique selon l'une quelconque des revendications **1** à **17,** dans laquelle l'huile est le TCM.

**19.** Émulsion ophtalmique cationique selon l'une quelconque des revendications **1** à **18,** comprenant du chlorure de benzalkonium comme agent cationique et du tyloxapol comme tensio-actif non-ionique.

**20.** Émulsion ophtalmique cationique selon l'une quelconque des revendications **1** à **19,** dans laquelle l'émulsion contient du chlorure de benzalkonium comme agent cationique et une combinaison de tyloxapol et de poloxamère.

**21.** Émulsion ophtalmique cationique selon l'une quelconque des revendications **1** à **20,** dans laquelle lesdites particules colloïdales ont une taille moyenne inférieure ou égale à 1 $\mu$m, avantageusement inférieure ou égale à 300 nm, plus avantageusement dans la gamme allant 100 à 250 nm.

**22.** Émulsion ophtalmique cationique type huile-dans-eau selon l'une quelconque des revendications **1** à **21,** dans laquelle l'émulsion satisfait aux exigences du Test A de stabilité du potentiel zêta tel que défini dans la description, dans laquelle le potentiel zêta est mesuré à T=0, l'émulsion est conservée à 80°C pendant 15 heures et mesurée à T=15 heures, l'émulsion satisfaisant aux exigences du Test A de stabilité du potentiel zêta quand la variation du potentiel zêta, delta A = $Z_{15h}$-$Z_0$, entre deux mesures, est inférieur à l'écart-type de mesures, préférablement inférieure à 10 mV.

**23.** Émulsion ophtalmique selon l'une quelconque des revendications **1** à **21,** qui satisfait aux exigences du Test B de stabilité du potentiel zêta tel que défini dans la description, dans laquelle le potentiel zêta $Z_0$ est mesuré à T=0, puis l'émulsion est conservée pendant 48 heures à 80°C et le potentiel zêta $Z_2$ est mesurée le jour 2, l'émulsion satisfaisant aux exigences du Test B de stabilité du potentiel zêta quand la variation du potentiel zêta, delta B = $Z_2$-$Z_0$, entre deux mesures est inférieur à l'écart-type de mesures, préférablement inférieure à 10 mV.

**24.** Émulsion ophtalmique selon l'une quelconque des revendications **1** à **21,** qui satisfait aux exigences du Test C de stabilité du potentiel zêta tel que défini dans la description, dans laquelle le potentiel zêta $Z_0$ est mesuré à T=0, puis l'émulsion est conservée pendant 7 jours à 80°C et le potentiel zêta $Z_7$ est mesurée le jour 7, l'émulsion satisfaisant aux exigences du Test C de stabilité du potentiel zêta quand la variation du potentiel zêta, delta C = $Z_7$-$Z_0$, entre deux mesures est inférieur à l'écart-type de mesures, préférablement inférieure à 10 mV.

**25.** Émulsion ophtalmique selon l'une quelconque des revendications **1** à **21,** qui satisfait aux exigences du Test D de stabilité du potentiel zêta tel que défini dans la description, dans laquelle le potentiel zêta $Z_0$ est mesuré à T=0, puis l'émulsion est conservée pendant 14 jours à 80°C et le potentiel zêta $Z_{14}$ est mesurée le jour 14, l'émulsion satisfaisant aux exigences du Test D de stabilité du potentiel zêta quand la variation du potentiel zêta, delta D = $Z_{14}$-$Z_0$, entre deux mesures est inférieur à l'écart-type de mesures, préférablement inférieure à 10 mV.

**26.** Émulsion ophtalmique selon l'une quelconque des revendications 1 à 25, cette émulsion était sous la forme de collyre, de pommade, de gel ophtalmique.

**27.** Émulsion ophtalmique selon l'une quelconque des revendications **1** à **25,** dans laquelle l'émulsion se trouve dans un dispositif d'administration sélectionné dans le groupe comprenant les lentilles, le patch oculaire, l'implant, l'insert.

**28.** Utilisation d'une émulsion ophtalmique cationique type huile-dans-eau selon l'une quelconque des revendications **1** à **27,** pour la préparation d'une composition ophtalmique pour le traitement de l'hypertension oculaire et/ou le traitement du glaucome.

Figure 1

Figure 2

# EP 1 937 212 B1

## REFERENCES CITED IN THE DESCRIPTION

### Patent documents cited in the description

- US 6011062 A
- US 5688819 A
- US 5849792 A
- US 4599353 A
- US 6007826 A
- WO 2005044276 A

### Non-patent literature cited in the description

- **C. Debbasch et al.** *Investigative Ophthalmology & Visual Science,* March 2001, vol. 42 (3
- **Klang et al.** *Pharm. Dev. Technology,* 2000, vol. 5, 521-532
- **Washington.** *Adv. Drug Deliv. Reviews,* 1996, vol. 20, 131-195
- **Tamilvanan et al.** *STP Pharma Sciences,* 2001, vol. 11, 421-426
- **Furrer et al.** *Eur. J. Pharm. Biopharm.,* 2002, vol. 53, 263-280